# EUROPEAN PATENT APPLICATION

(11) **EP 4 807 672 A1**
(43) Date of publication of application: **16.09.2026**
(21) Application number: 25163149.5
(22) Date of filing: 12.03.2025
(51) Int. Cl.: G06T 7/10

(54) **AUTO SEGMENTATION OF THALAMIC NUCLEI USING TRACK DENSITY IMAGES**

(71) Applicant: Siemens Healthineers AG, 91301 Forchheim (DE)
(72) Inventor: RAVI, C. A., Bangalore 560100 (IN); HUWER, Stefan, 91052 Erlangen (DE)
(74) Representative: HKW Intellectual Property PartG mbB

(57) **Abstract**

The invention relates to a computer-implemented method for training an artificial intelligence model for segmenting a thalamic nucleus in a radiology image, comprising obtaining a plurality of radiology images comprising a representation of thalamic nuclei, preprocessing the obtained plurality of radiology images, wherein the preprocessing comprises generating a plurality of track density images, TDI, based on the generated plurality of radiology images and training the artificial intelligence model based on at least a first portion of the generated plurality of TDIs.

## Description

The present invention relates to a computer-implemented method for training an artificial intelligence model for segmenting a thalamic nucleus in a radiology image, a respective trained artificial intelligence model, a computer-implemented method for segmenting a thalamic nucleus in a radiology image, an apparatus for training an artificial intelligence model for segmenting a thalamic nucleus in a radiology image and a computer-program product.

Medical imaging has provided a powerful tool for diagnosing abnormalities in a body, head and/or leg of a patient for many years and it has developed to represent a standard procedure in state-of-the-art medical diagnostics. Medical imaging made it possible to support a non-invasive diagnosis based on images that were captured of certain parts of a body of a patient. Such images may, e.g., be magnetic resonance images (MRIs).

The imaging quality of these imaging methods has drastically improved over the last decades supporting the accurate identification of ever decreasing abnormalities. The imaging quality has further improved such that it allows a precise capturing of, e.g., magnetic field information associated with parts of the body of the patient with an improved contrast ration (as compared to respective devices used decades ago) and suppressed blurring effects. These improvements in the imaging quality have advantageously contributed to a general improvement of medical imaging and has led to an increase in the constant demand for medical imaging even further.

However, even nowadays, the captured images (especially when it comes to, e.g., angiography images) are, to a wide extent, still analyzed manually by an experienced physician. This is time and resource intensive and may lead to high costs of medical imaging which, in turn, need to be compensated by already weakened healthcare systems and are accompanied by prolonged waiting times for the patients requiring a medical imaging-based diagnosis.

The detection of small anomalies in the body of a patient may play a dominant role when regions of the (human) brain need to be investigated. These small-scale structures can oftentimes not be identified in a reliable manner based on currently existing (mostly manual) methods of reviewing medical images.

This may be of relevance, if a structure of a thalamus is to be studied. The thalamus is a relay organ of the (human) brain that has been linked to several higher order neurological processes, such as controlling awareness, sleep, alertness and transmitting sensory and motor impulses to the cerebral cortex.

Numerous brain disorders, including multiple sclerosis, alcohol use disorder, essential tremor, schizophrenia, and Alzheimer's disease have been linked to the thalamus.

These pathologies may differentially impact individual thalamic nuclei. When doing deep brain surgery (DBS) to treat essential tremor, precise and patient-specific localization of nuclei help to reduce surgical targeting errors. One such nucleus is the Ventralis intermedius (Vim).

Due to weak intra-thalamic nuclear contrast, individual thalamic nuclei may not readily be apparent on standard T1 or T2 weighted MRI sequences such as Magnetization Prepared Rapid Gradient Echo (MP-RAGE) or Fast/Turbo Spin Echo (FSE/TSE).

Because of this, clinical applications such as DBS, focused ultrasound thalamotomy, have relied on standard atlases and awake physiologic confirmation of thalamic nuclei localization.

Therefore, the oftentimes required segmentation of thalamic nuclei in medical images may appear cumbersome (e.g., as the segmentation task may oftentimes be done manually) and may not always allow a segmentation with sufficient accuracy (in particular, if an atlas-based segmentation is applied, e.g., by means of a Morel atlas and/or a thalamic optimized multi-atlas segmentation (THOMAS) technique based on white-matter-nulled Magnetization Prepared - Rapid Gradient Echo (MP-RAGE)). What is more, the localization and segmentation of thalamic nuclei may appear difficult for novice users, making the segmentation more prone to errors with possible adverse effects on a health state of a patient.

Thus, it is an object of the present invention to provide an efficient segmentation procedure of thalamic nuclei in medical images.

According to a first aspect, a computer-implemented method for training an artificial intelligence model for segmenting a thalamic nucleus in a radiology image is suggested. The computer-implemented method may comprise obtaining a plurality of radiology images comprising a representation of thalamic nuclei and preprocessing the obtained plurality of radiology images, wherein the preprocessing comprises generating a plurality of track density images, TDI, based on the generated plurality of radiology images. Moreover, the computer-implemented method may comprise training the artificial intelligence model based on at least a first portion of the generated plurality of TDIs.

In some examples, the radiology images may comprise characteristics which may support the generation of a TDI based on a respective radiology image.

Thalamic nuclei may be referred to as clusters of densely packed neuronal cell bodies within a thalamus, a paired gray matter structure located in the center of the brain. These nuclei may serve as critical relay stations for sensory and motor information, integrating and modulating signals before transmitting them to the cerebral cortex. The thalamus may be divided into several groups of nuclei, including anterior, medial, and lateral nuclei, each with distinct functions and connections. These nuclei may play essential roles in processing sensory modalities, regulating consciousness, sleep, and alertness, and facilitating motor activity and emotional responses of a patient. The thalamic nuclei may play a crucial role for the proper functioning of the brain, acting as a gateway for information exchange between the periphery and the cerebral cortex.

The obtaining may comprise a retrieving of the plurality of medical images from a remote entity, such as, e.g., a remote database. The plurality of medical images may at least partially be obtained over an intranet. Additionally or alternatively, the plurality of medical images may at least partially be obtained over the internet.

A TDI may be referred to as a neuroimaging technique based on diffusion magnetic resonance imaging (dMRI) that may generate high-resolution images of white matter of the brain by analyzing fiber tracts (e.g., nerve bundles). This method may utilize a density, a measure of how many tracts per voxel, of fiber tracts passing through the brain to produce intra-voxel information that exceeds the original resolution of the dMRI data and thus can depict the thalamic nuclei much better than the standard contrasts.

This may facilitate an efficient training of an artificial intelligence model which may be configured to subsequently segment thalamic nuclei. Such a trained artificial intelligence model may support an efficient, simplified and automated segmentation of thalamic nuclei in medical images. Based thereon, an improved diagnosis of potential neuronal diseases, which may arise from malformations of the thalamic nuclei, may be supported.

According to an embodiment, the obtaining of the plurality of radiology images may comprise obtaining the plurality of radiology images for patients of different age groups and ethnicities, preferably at least for 250 patients.

In some examples, the age groups may be provided such that patients in an age range of 3 to 99 years may be represented in the obtained plurality of radiology images. In some examples, patients in an age range of 18 to 99 years may be represented in the obtained plurality of radiology images. In some examples, patients in an age range of 18 to 67 years may be represented in the obtained plurality of radiology images. In some examples, patients of another age group may be represented in the obtained plurality of radiology images.

In some examples, the plurality of radiology images may be obtained for more than 300 patients, more than 500 patients or more than 1000 patients. In some examples, the plurality of radiology images may be obtained for any other suitable number of patients.

Obtaining the plurality of radiology images from patients of different age groups and ethnicities may support an obtaining of a generic set of training data which may be representative for a wide range of different patients. Consequently, an improved and advanced trained artificial intelligence model may be provided which may allow a more accurate segmentation of thalamic nuclei in radiography images.

According to a further embodiment, the computer-implemented method may further comprise validating the trained artificial intelligence model with at least a second portion of the plurality of TDIs, wherein the second portion of the TDIs is not used for training the artificial intelligence model.

The validation may be referred to as evaluating the artificial intelligence model's performance on a validation dataset, which may be different from the training dataset.

In some examples, the first portion of the TDIs and the second portion of the TDIs, when combined, may result in the original total set of generated TDIs.

In some examples, 250 radiography images may be obtained and transformed into 250 respective TDIs. In some examples, the first portion of the TDIs may comprise 200 radiography images whereas the second portion of the TDIs may comprise 50 radiography images.

The validation may comprise calculating a dice score or a similarity matrix to quantify an accuracy of the trained artificial intelligence model in providing segmented thalamic nuclei in a radiography image (and/or a TDI).

This process may ensure that the artificial intelligence model generalizes effectively and has not overfit the training data set, allowing for adjustments to hyperparameters and model architecture to optimize performance. The validation dataset may provide an unbiased assessment of the model's ability to make accurate predictions on unseen data, which may be beneficial for ensuring the model's reliability and robustness in real-world applications.

According to a second aspect, a computer-implemented method for segmenting a thalamic nucleus in a radiology image is suggested. The computer-implemented method may comprise obtaining a radiology image comprising a representation of the thalamic nucleus and preprocessing the obtained radiology image, wherein the preprocessing comprises generating a track density image, TDI, based on the obtained radiology image. Moreover, the computer-implemented method may comprise providing the generated TDI to a trained artificial intelligence model, wherein the trained artificial intelligence model is trained according to the computer-implemented method as disclosed herein and segmenting, using the trained artificial intelligence model, the thalamic nucleus in the TDI image.

In some examples, a single radiology image may be processed by the computer-implemented method for segmenting the thalamic nucleus. In some examples, a plurality of radiology images may be processed by the computer-implemented method for segmenting the thalamic nucleus.

In some examples, a single thalamic nucleus may be segmented in the radiography image (and/or the TDI). In some examples, a plurality of thalamic nuclei may be segmented in the radiography image.

This may facilitate a segmentation on the fly using a trained artificial intelligence model (e.g., based on a CNN). Moreover, the segmentation may be patient-specific as it may be based on a radiology image of the patient and based on the generically trained artificial intelligence model. This may facilitate a high accuracy and a fully automated segmentation, i.e., a segmentation which does not require a manual intervention by a physician or any other stakeholder. Still remaining accuracy gaps (e.g., a difference between a current segmentation accuracy and a desired segmentation accuracy) may be filled by repeatedly training a respective artificial intelligence model (e.g., a CNN).

According to an embodiment, the obtained radiology image may be a diffusion weighted image, DWI, preferably using a high angular resolution diffusion imaging, HARDI, sequence.

In DWIs, regions with high water diffusion, such as cerebrospinal fluid, may appear dark due to significant signal attenuation, while areas with restricted diffusion, like ischemic brain tissue, may appear bright. This property may make DWIs particularly useful for diagnosing conditions such as acute strokes and tumors, where changes in water diffusion can indicate early pathological changes. Additionally, DWIs may be used to create apparent diffusion coefficient (ADC) maps, which may quantify the diffusion coefficient and help differentiate between diffusion and other effects like perfusion, providing a more accurate assessment of tissue characteristics.

HARDI may be capable of resolving intravoxel heterogeneity, meaning it can distinguish between multiple fiber populations within a single voxel, which is a limitation of diffusion tensor imaging (DTI)s. By reconstructing the orientation distribution function (ODF), HARDI may provide a more comprehensive understanding of tissue microstructure and is particularly useful for diffusion tractography. This technique may enable the tracking of white matter tracts through regions of crossing fibers, where DTI may often fail to provide accurate results. Overall, HARDI may enhance the accuracy and resolution of diffusion imaging, offering valuable insights into brain connectivity and micro architecture.

This may allow capturing a type of radiography images, which carries tailored information for subsequently deriving a TDI which, in turn, may be used for an automated segmentation of thalamic nuclei.

According to a further embodiment, the plurality of radiology images may be a plurality of diffusion weighted images, DWI, preferably using a high angular resolution diffusion imaging, HARDI, sequence.

A DWI may be referred to as a type of MRI which may generate contrast in a radiography image based on a random Brownian motion of water molecules within tissues. This technique may use specific MRI sequences that apply additional gradient pulses to the standard T2-weighted pulse sequence, making it sensitive to the diffusion of water molecules in different directions.

HARDI may be referred to as an advanced diffusion-weighted imaging technique that may provide detailed information about the orientation and distribution of white matter tracts in the brain.

This may allow capturing of a type of radiography images which carries tailored information for subsequently deriving a TDI which, in turn, may be used for an automated segmentation of thalamic nuclei.

According to a further embodiment, obtaining the plurality of radiology images may further comprise obtaining the plurality of radiology images, preferably for at least 45 diffusion directions, preferably with a diffusion-related b-value of at least 3000 s/mm².

A diffusion direction may be referred to as a specific orientation in which the diffusion of water molecules in the body of a patient is measured. These directions may be determined by the application of magnetic field gradients during an MRI sequence. The gradients may be applied in various directions to assess how water molecules move in those specific orientations, which may be crucial for understanding a tissue microstructure and detecting anisotropic diffusion. Brownian motion of water molecules may be restricted by a nerve fiber enclosing myelin sheaths and may thus be larger along the nerval fiber detection than across it. From these restrictions an indirect model of fiber orientations within a voxel can be built. The model may be improved the more diffusion directions at higher b-values are acquired.

A capturing of DWI sequences may apply diffusion-sensitizing gradients in at least three orthogonal directions (x, y, and z). In some examples, to capture anisotropic information in a voxel, at least 6 gradient directions may be required. The higher a number of directions and the lower an angular distance between neighboring detections, the better the capabilities to explain a crossing of fiber structures at lower angles of a gradient may be.

The b-value may be representative for a degree of diffusion weighting which may define a strength, duration, and separation time of gradient pulses used for capturing the medical images. Higher b-values may be representative for an increase of the sensitivity to diffusion, allowing for better detection of differences in water mobility.

This may allow for the calculation of parameters such as fractional anisotropy and mean diffusivity, providing insights into tissue architecture and orientation. What is more, the obtained detailed information about diffusion directions may be used for accurately mapping white matter tracts and understanding brain connectivity.

According to a further embodiment, the preprocessing may further comprise modeling a diffusion process depicted in the DWI by means of a spherical harmonic function, wherein the spherical harmonic function is, preferably at least of order 8.

The spherical harmonic function may be used in DWIs to efficiently model and analyze the diffusion distribution in, e.g., three dimensions. The spherical harmonic function may provide a precise representation of diffusion directions and strengths, which may be advantageous for capturing complex diffusion patterns in brain tissue such as the thalamic nuclei.

In some examples, the number of diffusion directions may depend on the order of the spherical harmonic function. In some examples, if the order of the spherical harmonic function is 8, the minimal number of diffusion directions may be 45. If the order of the spherical harmonic function is 10, the minimal number of diffusion directions may be 66. If the order of the spherical harmonic function is 12, the minimal number of diffusion directions may be 91. If the order of the spherical harmonic function is 14, the minimal number of diffusion directions may be 120.

This may allow a modeling of a diffusion distribution as spherical harmonics may support a description of the diffusion distribution within a voxel by modeling diffusion in various directions. This is particularly useful in areas where multiple fiber tracts intersect. Moreover, the spherical harmonic function may enhance a resolution of the information derivable from the TDI. The use of spherical harmonics can improve the resolution of diffusion data by providing a more detailed representation of diffusion directions. Moreover, the spherical harmonic function may support fiber tracking as it may enable a more precise modeling of fiber orientations. This may be important for reconstructing nerve fibers in the thalamic nuclei under investigation. Moreover, the spherical harmonic function may enable an anisotropy visualization. That is, the spherical harmonic function may support a visualization of anisotropic diffusion in tissues, which may be seen crucial for understanding the thalamic nuclei in detail.

According to a further embodiment, the preprocessing may further comprise setting a field of view, FOV, in the obtained plurality of radiology images to minimize a TDI generation time.

The FOV may define a region of interest (ROI) in the radiology image. The FOV may define a region of a radiology image which may subsequently be used (exclusively) for the generation of a TDI.

By effectively limiting the radiology image to an ROI only, the time required for generating a TDI may be decreased and the preprocessing may be performed in a more efficient manner.

According to a further embodiment, the TDI may comprise at least 15 million tracks, preferably with an isotropic voxel resolution of 0.3mm to 0.4mm voxel side length.

A track in a TDI may be referred to as a computationally reconstructed path representing a trajectory of a nerve fiber bundle through the brain of the respective patient. These tracks may be derived from the analysis of fiber tract density, which may be calculated from DWI data, providing detailed insights into brain connectivity and white matter structure of the brain of the patient.

In some examples, the TDI may at least comprise 20 million tracks, at least 30 million tracks, at least 50 million tracks or more tracks. In some examples, the TDI may also comprise less than 15 million tracks such as less than 10 million tracks, less than 5 million tracks or less than 1 million tracks.

By generating the TDI in a way that it at least comprises 15 million tracks and such that it preferably has a resolution of 0.3mm to 0.4mm, a high resolution TDI may be provided that may efficiently support a subsequent demarcation of thalamic nuclei in the respective radiography images.

According to a further embodiment, the preprocessing may further comprise segmenting thalamic nuclei in the generated plurality of TDIs, preferably manually by a radiologist.

The segmenting of the thalamic nuclei may comprise a (manual) demarcation of one or more thalamic nuclei in the radiography image (and the TDI, respectively).

A manual segmentation may be referred to as a segmentation that is carried out by a radiologist, i.e., a radiologist may, based on his/her knowledge and/or based on literature, define which segment of the radiology image (or the TDI) corresponds to which anatomical region of the brain.

In an example, one or more of the following thalamic nuclei may be (manually) segmented: ventral anterior nucleus (VA, involved in motor function and projects to the premotor cortex), ventral lateral nucleus (VL, important for motor function and projects to the motor cortex), ventral posterior nucleus (VP, serves as a relay station for sensory signals, particularly for proprioception and touch), anterior thalamic nuclei (functionally associated with the limbic system and important for emotional processes), medial thalamic nuclei (involved in higher cognitive functions and projects to the prefrontal cortex), lateral geniculate body (a crucial relay station in the visual pathway), medial geniculate body (a key relay station in the auditory pathway), pulvinar (involved in perception, memory, and language), intralaminar nuclei (located in the internal medullary lamina of the thalamus, these nuclei play a role in the non-specific activation of the cortex), reticular nucleus of the thalamus (functions as a filter for the information passing through the thalamus and inhibits the specific thalamic nuclei).

The (manual) segmenting of the thalamic nuclei may provide a (manually) annotated set of radiography images (and/or TDIs) which may be used for training a respective artificial intelligence model to segment thalamic nuclei.

According to a further embodiment, the artificial intelligence model may comprise a convolutional neural network, CNN, in a U-net architecture.

A CNN may be referred to as a neural network that consists of multiple layers, including convolutional layers, pooling layers, and fully connected layers. The convolutional layers may apply filters to small regions of the input radiography image, scanning the data in a sliding window fashion to generate feature maps that capture local patterns and structures. Moreover, pooling layers may be implemented to reduce the spatial dimensions of these feature maps, effectively downsampling the data (i.e., the radiography images) to retain only the most important information. Moreover, fully connected layers may be applied to interpret these features to make predictions or classifications.

A U-Net architecture may be referred to as a type of convolutional neural network designed for image segmentation tasks. It may comprise a contracting path, referred to as an encoder, and an expansive path, referred to as a decoder. The encoder involves a series of convolutional layers followed by max pooling operations, which may reduce spatial dimensions while increasing a number of feature channels. Such a process may capture context information from the input radiology image.

The decoder path may involve upsampling operations followed by convolutional layers, which may restore a spatial resolution of feature maps. In some examples, the U-Net may comprise skip connections between the encoder and decoder, allowing the network to preserve high-resolution spatial information from the early layers and combine it with the semantic information from the deeper layers.

Using a U-net architecture may allow for a precise segmentation by leveraging both spatial and contextual information, making it highly effective for the segmentation of thalamic nuclei in radiography images and derived TDIs, respectively.

According to a third aspect, a trained artificial intelligence model for segmenting thalamic nuclei in a radiology image is suggested. The trained artificial intelligence model may have been trained according to the computer-implemented method as disclosed herein.

In some examples, the segmenting of thalamic nuclei may effectively be performed based on a TDI which may have been derived from the radiology image. Therefore, the segmenting may be referred to as a segmenting of thalamic nuclei in radiology images based on intermediately derived TDIs.

The trained artificial intelligence model may be implemented to segment thalamic nuclei in TDIs of a patient who has newly arrived at a medical facility.

In some examples, the artificial intelligence model may be registered with a radiology image of the patient to correctly identify thalamic nuclei of interest which may thus be patient specific and may render the segmentation patient specific.

Thus, a trained artificial intelligence model may be provided which may provide versatile implementation options for a subsequent segmenting of thalamic nuclei in a radiography image.

According to a fourth aspect, a computer-implemented apparatus for training an artificial intelligence model for segmenting a thalamic nucleus in a radiology image is suggested. The computer-implemented apparatus may comprise an obtaining unit for obtaining a plurality of radiology images comprising a representation of thalamic nuclei and a preprocessing unit for preprocessing the obtained plurality of radiology images, wherein the preprocessing comprises generating a plurality of track density images, TDI, based on the generated plurality of radiology images. Moreover, the computer-implemented apparatus may comprise a training unit for training the artificial intelligence model based on at least a first portion of the generated plurality of TDIs.

The generating of TDIs may provide an advantage over a solely, e.g., MRI-based segmentation of thalamic nuclei. Routine MRI may not be able to distinguish between thalamic nuclei. Hence, targeting is based on anatomic atlas-based coordinates. So, there will be less consideration for individual variability. In a thalamic region, the white matter tracts may be densely populated. Each thalamic nucleus may have its own tract bundles which may connect to other cortex regions of the brain. In contrast, TDI (maps) derived from probabilistic diffusion tractography, may provide good internal contrast between the respective thalamic nuclei.

According to a fifth aspect, a computer program product is suggested that comprises instructions which, when the program is executed by a computer, cause the computer to carry out the computer-implemented method as disclosed herein.

A computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

According to a further embodiment, the segmenting may comprise segmenting a plurality of thalamic nuclei in the TDI image.

In some examples, the segmenting may comprise segmenting one or more thalamic nuclei of the group of thalamic nuclei as outlined above.

By segmenting a plurality of thalamic nuclei in the TDI image, a wide range of regions of a thalamus may be demarcated in a radiography image. This may efficiently support a subsequent diagnosis of potentially present neural diseases.

In an embodiment, the computer-implemented apparatus may comprise an execution unit for executing the computer-implemented method for training an artificial intelligence model for segmenting a thalamic nucleus in a radiology image as outlined above.

According to a sixth aspect, a computer-implemented apparatus for segmenting a thalamic nucleus in a radiology image is suggested. The computer-implemented apparatus may comprise an obtaining unit for obtaining a radiology image comprising a representation of the thalamic nucleus and a preprocessing unit for preprocessing the obtained radiology image, wherein the preprocessing comprises generating a track density image, TDI, based on the obtained radiology image. The computer-implemented apparatus may further comprise a providing unit for providing the generated TDI to a trained artificial intelligence model, wherein the trained artificial intelligence model is trained according to the computer-implemented method as disclosed above. Moreover, the computer-implemented apparatus may further comprise a segmenting unit for segmenting, using the trained artificial intelligence model, the thalamic nucleus in the TDI image.

In an embodiment, the computer-implemented apparatus may comprise an execution unit for executing the computer-implemented method for segmenting a thalamic nucleus in a radiology image as outlined above.

According to a seventh aspect, a system for segmenting a thalamic nucleus in a radiology image is suggested. The system may comprise an execution unit for executing the computer-implemented method for segmenting a thalamic nucleus in a radiology image as discussed above. Moreover, the system may comprise an execution unit for executing the computer-program product as discussed above.

In some examples, the computer-implemented methods as set out herein and the computer-implemented apparatuses as set out herein, is not exclusively applied to the segmentation of thalamic nuclei but may also be used for an (automated) segmentation of structures of the brain stem.

Even though some embodiments are described in isolation from each other, they may nevertheless also be combined with each other.

It is further noted that aspects directed to a plurality of radiology images may also cover the example in which only single radiography image is used (and vice versa).

The embodiments and features described with reference to the apparatus of the present invention apply, mutatis mutandis, to the method of the present invention and vice versa.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features and advantages of the present invention will become apparent from the subsequent description and dependent claims, taken in conjunction with the accompanying drawings, in which:
- Figs. 1A and 1B: show examples of a manual segmentation according to a Morel Atlas;
- Fig. 2: shows an exemplary comparison between a THOMAS-based and an atlas-based segmentation;
- Fig. 3: shows an exemplary view of a human brain taken in an axial plane;
- Fig. 4: shows an exemplary TDI;
- Fig. 5: shows an exemplary (manual) annotation of thalamic nuclei;
- Fig. 6: shows an exemplary illustration of a U-net architecture;
- Fig. 7: shows an exemplary workflow for a method for training an artificial intelligence model for segmenting a thalamic nucleus in a radiology image;
- Fig. 8: shows an exemplary workflow for segmenting a thalamic nucleus in a radiology image;
- Fig. 9: shows an illustration of an apparatus for training an artificial intelligence model for segmenting a thalamic nucleus in a radiology image; and
- Fig. 10: shows an illustration of an apparatus for segmenting a thalamic nucleus in a radiology image.

In the Figures, like reference numerals designate like or functionally equivalent elements, unless otherwise indicated.

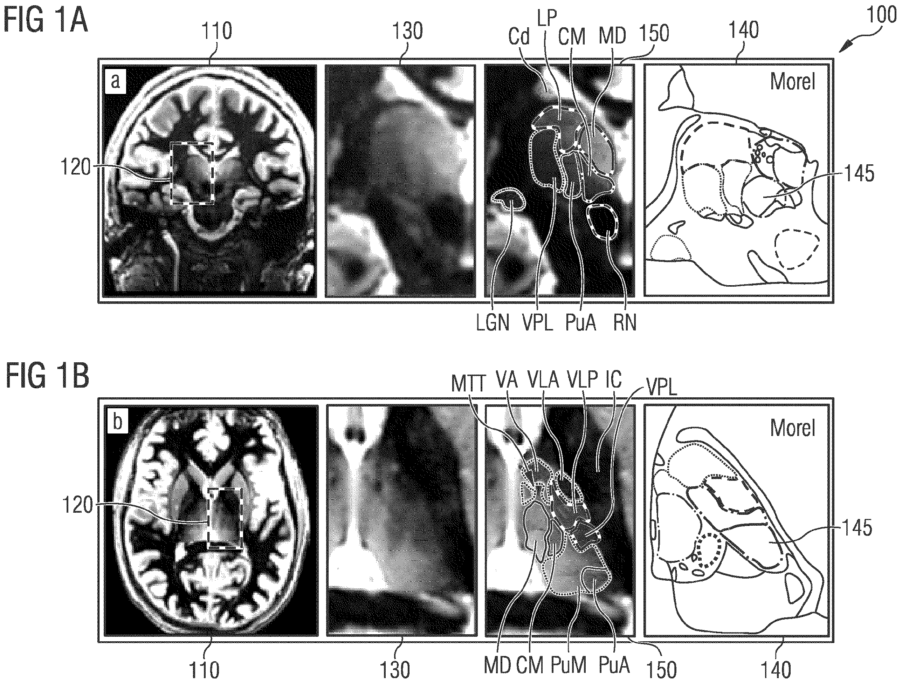

Figs. 1A and 1B show examples of a manual segmentation 100 according to a Morel Atlas.

More specifically, Fig. 1A shows a manual segmentation in a coronal plane of a body of a patient.

Segment 110 of Fig. 1A shows a human brain in a coronal plane. Segment 120 highlights a region of interest 120 that is shown in an enlarged view in segment 130 of Fig. 1A.

Segment 140 of Fig. 1A shows exemplary cutouts 145 of thalamic regions of the Morel Atlas matching the portion of the human brain that has been defined as the region of interest 120.

Segment 150 of Fig. 1A shows an overlay view of the region of interest 130 and the respective corresponding cutouts 145 of the Morel Atlas to segment the thalamic nuclei depicted in the region of interest 120.

Fig. 1B shows a manual segmentation of thalamic nuclei in an axial plane.

Segment 110 of Fig. 1B shows a human brain in the axial plane. Segment 120 highlights a region of interest 120 that is shown in an enlarged view in portion 130 of Fig. 1B.

Segment 140 of Fig. 1B shows exemplary cutouts 145 of thalamic regions of the Morel Atlas matching the portion of the human brain that has been defined as the region of interest 120.

Segment 150 of Fig. 1B shows an overlay view of the region of interest 130 and the respective corresponding cutouts 145 of the Morel Atlas to segment the thalamic nuclei depicted in the region of interest 120.

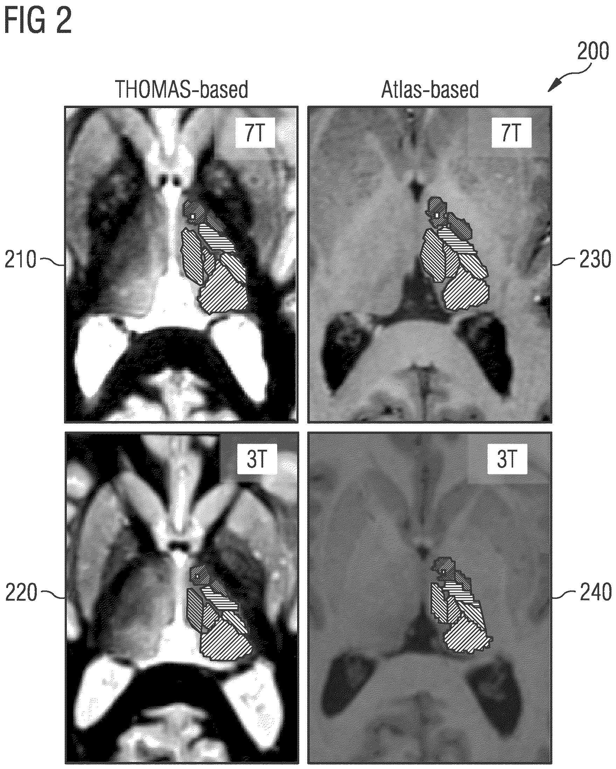

Fig. 2 depicts an exemplary comparison 200 between a THOMAS-based (segments 210 and 220) and an atlas-based (segments 230 and 240) segmentation of thalamic nuclei of a multiple sclerosis patient for a magnetic field of 7T (top row, portions 210 and 230) and for a magnetic field of 3T (bottom row, portions 220 and 240) during MRI-based image capture.

As derivable from segments 210 - 240, an applied magnetic field of 7T (and thus a higher magnetic field) may generally lead to an improved imaging quality and thus to a more accurate segmentation of thalamic nuclei. This may be derivable from Fig. 2 and in particular from segments 210 and 230 showing more sharpened demarcation lines surrounding the respective thalamic nuclei as compared to segments 220 and 240.

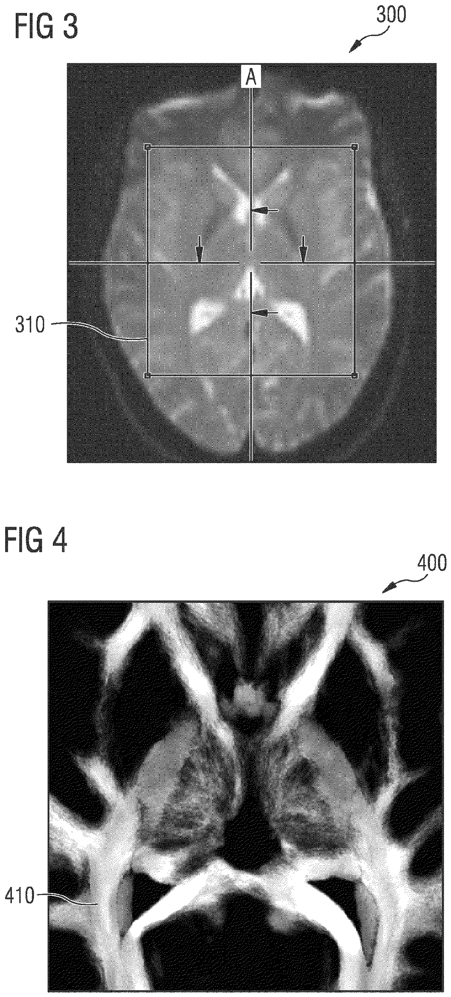

Fig. 3 depicts an exemplary view 300 of a human brain taken in an axial plane.

At least a portion of the human brain depicted in Fig. 3 is selected as a region of interest 310 acting as a FOV as described above. The FOV may support an efficient and faster generation of TDIs.

Fig. 4 depicts an exemplary TDI 400 which has been generated based at least in part on a defined FOV (e.g., the FOV 310 as described with reference to Fig. 3, above).

The TDI 400 depicts a plurality of tracks 410 which indicate nerves in the human brain under investigation and depicted in the defined region of interest 310.

The TDI 400 may be based on computing numerous fiber tracts in a representation of the (human) brain (e.g., in the FOV), which may represent an orientation and distribution of nerve fibers. Afterwards, the number of tracts passing through each voxel may be counted to determine a density of fibers and also the orientation of fibers. Finally, these density values may be used to create an image that displays the distribution, orientation and density of fiber tracts in the brain.

Generally, one of the key advantages of TDIs (such as the TDI 400) may be seen in its ability to achieve high resolution imaging, leveraging long-range information from the fiber tracts to produce images with higher resolution than the original dMRI data. This may allow for a visualization of fine anatomical structures in the brain that are not visible with conventional methods.

TDIs (such as the TDI 400) may be particularly useful for studying white matter in the brain and may aid in the diagnosis of neurodegenerative diseases. The technique may offer high anatomical contrast and can adjust spatial resolution and signal-to-noise ratio based on the desired image resolution and the number of generated fiber tracts.

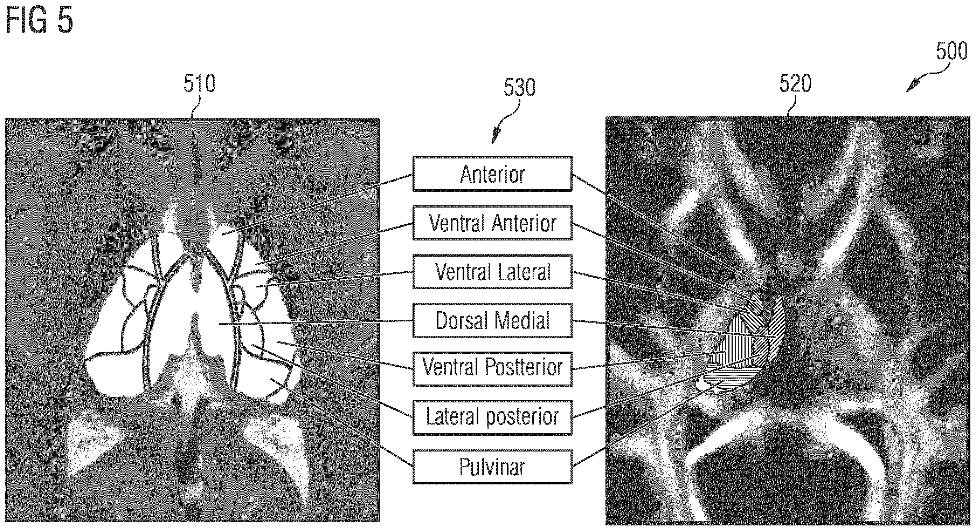

Fig. 5 depicts an exemplary (manual) annotation 500 of thalamic nuclei (according to a respective atlas, such as, e.g., the Morel Atlas) in a radiography image, notably a DWI 510 and a TDI 520.

The annotation 500 may be used to annotate obtained radiography images (e.g., TDI 520) for a subsequent training of an artificial intelligence model which may be used for segmenting a thalamic nucleus in a respective radiology image.

The annotation 500 may be based on obtaining potential thalamic nuclei 530 from a respective atlas (e.g., the Morel Atlas). The thalamic nuclei 530 may, e.g., comprise one or more of an anterior region, a ventral anterior region, a ventral lateral region, a dorsal medical region, a ventral posterior region, a lateral posterior region and/or a pulvinar region.

The potential thalamic nuclei 530 may manually be assigned to respective regions of the DWI 510 and/or the TDI 520 such that an annotated DWI 510 and/or an annotated TDI 520 is obtained.

This may facilitate subsequent (supervised) learning as part of the training of a respective artificial intelligence model.

In some examples, it may be possible to use 256 flow directions without averaging which may achieve similar image quality as compared to acquiring 4 averages of 64 directions out of which 1 or 2 averages may be saved by applying the deep-learning reconstruction as set out herein. This may result in a typical scan time of approximately 5-8 minutes in a clinical scenario for the acquisition of a DWI.

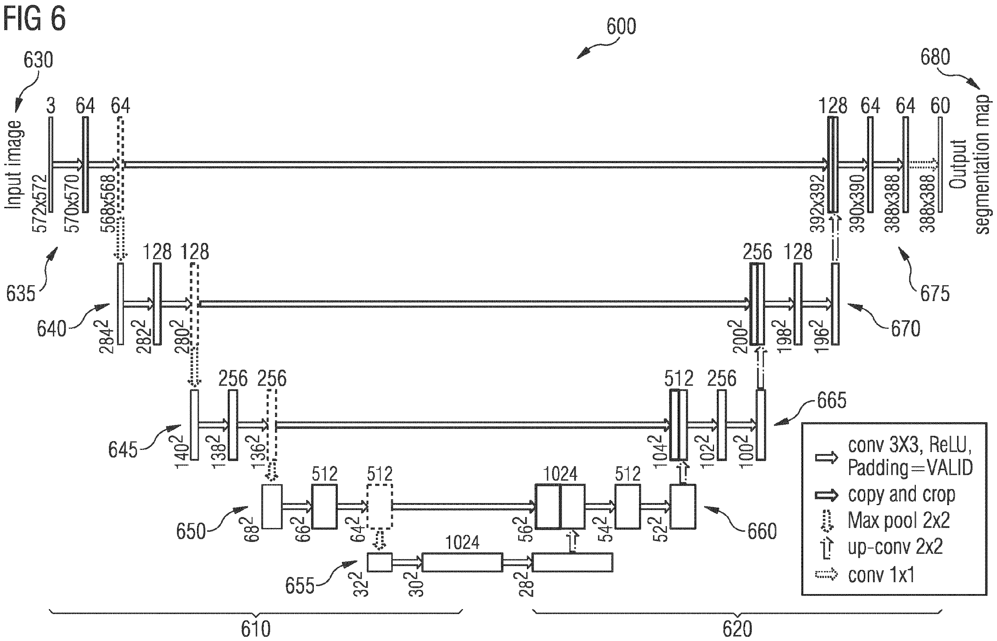

Fig. 6 shows an exemplary illustration of a U-net architecture 600 as it may be implemented as the artificial intelligence model for segmenting a thalamic nucleus in a radiology image. Alternatively, the U-net architecture 600 may be comprised by an artificial intelligence model for segmenting a thalamic nucleus in a radiology image.

It is emphasized that the U-net architecture 600, described with reference to Fig. 6, is not to be seen as exclusive for implementing a U-net architecture for segmenting a thalamic nucleus in a radiology image. Other implementations of a U-net architecture, not expressly set out herein, may also be possible.

The exemplary U-net architecture 600 may be used for both training an artificial intelligence model and for applying the trained artificial intelligence model for segmenting a thalamic nucleus in a radiology image.

The (rectangular) boxes depicted in Fig. 6 represent respective multi-channel feature maps.

The U-net architecture may comprise an encoder section 610 and a decoder section 620 which, in synergy, are configured to process an input image (i.e., a radiology image) 630 and to extract features from the input image 630 which can be used for segmenting a thalamic nucleus in the input image 630.

The encoder section 610 may comprise encoder steps 635 - 655, which each contribute to a dimension reduction of the input image 630.

In a first encoder step 635, the input image 630, e.g., with a resolution of 572 x 572 pixels may undergo a first convolution 1x1 procedure. This first convolution may be based on three channels. Subsequently, a 3x3 convolution, followed by a ReLu activation function (padding = valid) may be performed. The respective convolution may be based on 64 channels. This convolution may lead to a reduction of the resolution of the processed input image 630 to 570 x 570 pixels. Subsequently, another 3x3 convolution may be executed, based on 64 channels. This convolution may lead to a reduction of the resolution of the input image 630 to 568x568 pixels.

The first encoder step 635 may be followed by a second encoder step 640. A max pool layer of dimension 2x2 may be implemented between the first encoder step 635 and the second encoder step 640 which may lead to a further dimension reduction of the input image 630 to 284x284 pixels.

The second encoder step 640 may comprise a first convolution procedure of dimension 1x1 and based on 128 channels. This may be followed by a convolution of dimension 3x3 (ReLu, padding = valid), and a dimension reduction of the input image 630 to 282x282 pixel. This convolution may be followed by an additional convolution which may be implemented as the aforementioned convolution effectively leading to a dimension reduction of the input image 630 to 280x280 pixel.

The second encoder step 640 may be followed by a third encoder step 645. The third encoder step 645 may be implemented identical to the second encoder step 640. The second and third convolution of the third encoder step 645 may be based on 256 channels. After the third convolution of the third encoder step 645, the input image 630 may possess a dimensioning of 136x136 pixels.

The third encoder step 645 may be followed by a fourth encoder step 650. The fourth encoder step 650 may be configured identical to the third encoder step 645. The second and third convolution of the fourth encoder step 650 may be based on 512 channels. After the third convolution of the fourth encoder step 650, the input image 630 may possess a dimensioning of 64x64 pixels.

The fourth encoder step 650 may be followed by a fifth encoder step 655. The fifth encoder step 655 may be connected to the fourth encoder step 650 by means of a max pooling 2x2 procedure.

The fifth encoder step 655 may be initiated by a convolution of dimension 1x1 (512 channels, 32x32 pixels), followed by a convolution 3x3 (ReLu, padding = valid) which is accompanied by a reduction of the size of the input image 630 to 30x30 pixels. Subsequently, a convolution 3x3 (ReLu, padding = valid) may be executed (1024 channels) effectively decreasing the dimension of the input image 630 to 28x28 pixels.

This final convolution may terminate the encoder 610. Subsequently, the decoder 620 may reconstruct the encoded input image 630.

The operation of the decoder 620 may be initiated by a first up-convolution of dimension 2x2, effectively leading to an image dimension of 56x56 pixels. This may lead to the first decoder step 660.

In some examples, the underlying feature map may be copied.

Subsequently, another convolution 3x3 (ReLu, padding = valid) may be executed based on 1024 channels effectively increasing the size of the image to 54x54 pixels.

Another convolution of dimension 3x3 (ReLu, padding = valid) may be executed leading to an image size of 52x52 pixels.

An up-convolution of dimensions 2x2 may subsequently be executed, effectively leading to a size of 104x104 pixels of the (processed) input image 630. This may lead to the second decoder step 665.

In some examples, the underlying feature map may be copied.

Subsequently, a convolution of dimension 3x3 (ReLu, padding = valid) may be executed (based on 512 channels) which may lead to an image size of 102x102 pixels. This convolution may be followed by another identical convolution (based on 256 channels), yielding an image size of 100x100 pixels.

Subsequently, another up-convolution of dimension 2x2 may be executed, increasing the image size to 200x200 pixels and defining the third decoder step 670.

In some examples, the underlying feature map may be copied.

Afterwards, a convolution of dimension 3x3 (ReLu, padding = valid) may be executed (based on 256 channels) effectively leading to an image size of 198x198 pixels.

Another convolution may be executed (which may be based on 128 channels), effectively leading to an image size of 196x196 pixels.

The third decoder step 670 may be followed by a fourth decoder step 675 via an up-convolution of dimension 2x2 leading to an image size of 392x392 pixels.

In some examples, the underlying feature map may be copied.

Subsequently, a convolution of dimension 3x3 (ReLu, padding = valid) may be executed (based on 128 channels), leading to an image size of 390x390 pixels. This may be followed by another (identical) convolution (based on 64 channels), effectively leading to an image size of 388x388 pixels.

Afterwards a convolution of dimension 1x1 may be executed (e.g., based on 60 channels) leading to an image size of 388x388 pixels.

This image may be output as output segmentation map 680.

In some examples, the U-net architecture 600 may comprise skip connections between the first encoder step 635 and the fourth decoder step 680. Further skip connections may exist in between the second encoder step 640 and the third decoder step 670, between the third encoder step 645 and the second decoder step 665 and between the fourth encoder step 650 and the first decoder step 660.

In some examples, an Adam optimizer may be used to train the artificial intelligence network represented by or comprising the U-net architecture 600. In some examples, the learning rate may be 10⁻⁴, the batch size may be 8, the training may occur in 1000 epochs and a dice function may be used as a loss function.

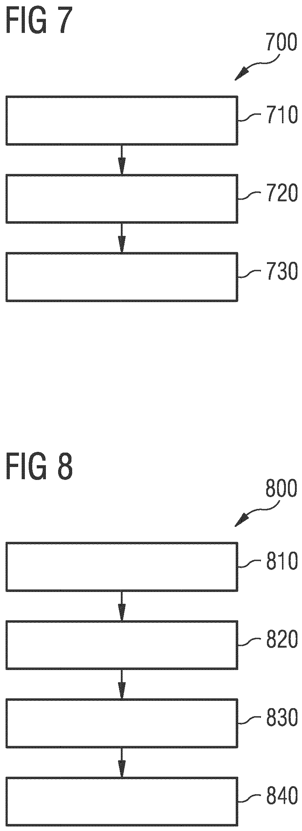

Fig. 7 depicts a workflow for a method 700 for training an artificial intelligence model for segmenting a thalamic nucleus in a radiology image.

In step 710, an obtaining of a plurality of radiology images comprising a representation of thalamic nuclei is executed.

In step 720, a preprocessing of the obtained plurality of radiology images is executed, wherein the preprocessing comprises generating a plurality of track density images, TDI, based on the generated plurality of radiology images.

In step 730, a training of the artificial intelligence model based on at least a first portion of the generated plurality of TDIs is executed.

Fig. 8 depicts a workflow for segmenting a thalamic nucleus in a radiology image.

In step 810, an obtaining of a radiology image comprising a representation of the thalamic nucleus is executed.

In step 820, a preprocessing of the obtained radiology image is executed, wherein the preprocessing comprises generating a track density image, TDI, based on the obtained radiology image.

In step 830, a providing of the generated TDI to a trained artificial intelligence model is executed, wherein the trained artificial intelligence model is trained according to the computer-implemented method as set out herein (e.g., according to the computer-implemented method described with reference to workflow 700).

In step 840, a segmenting of the thalamic nucleus in the TDI image is performed using the trained artificial intelligence model.

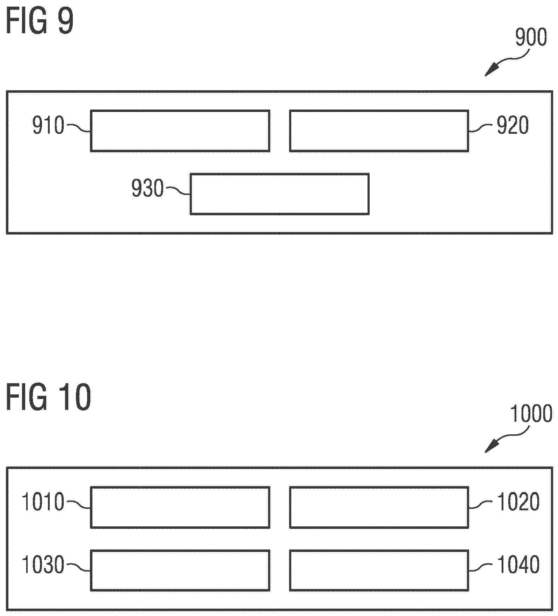

Fig. 9 shows an illustration of an apparatus 900 for training an artificial intelligence model for segmenting a thalamic nucleus in a radiology image. The apparatus 900 may comprise an obtaining unit 910, a preprocessing unit 920 and a training unit 930.

The obtaining unit 910 may be configured to obtain a plurality of radiology images comprising a representation of thalamic nuclei.

The preprocessing unit 920 may be configured to preprocess the obtained plurality of radiology images, wherein the preprocessing comprises generating a plurality of track density images, TDI, based on the generated plurality of radiology images.

The training unit 930 may be configured to train the artificial intelligence model based on at least a first portion of the generated plurality of TDIs.

Fig. 10 shows an illustration of an apparatus 1000 for segmenting a thalamic nucleus in a radiology image. The apparatus 1000 may comprise an obtaining unit 1010, a preprocessing unit 1020, a providing unit 1030 and a segmenting unit 1040.

The obtaining unit 1010 may be configured to obtain a radiology image comprising a representation of the thalamic nucleus.

The preprocessing unit 1020 may be configured to preprocess the obtained radiology image, wherein the preprocessing comprises generating a track density image, TDI, based on the obtained radiology image.

The providing unit 1030 may be configured to provide the generated TDI to a trained artificial intelligence model, wherein the trained artificial intelligence model is trained according to the computer-implemented method as set out herein (e.g., based on the computer-implemented method described with reference to workflow 700, above).

The segmenting unit 1040 may be configured for segmenting, using the trained artificial intelligence model, the thalamic nucleus in the TDI image.

Although the present invention has been described in accordance with preferred embodiments, it is obvious for the person skilled in the art that modifications are possible in all embodiments.

### REFERENCE NUMERALS

- 100: manual segmentation according to Morel Atlas
- 110: portion
- 120: portion
- 130: region of interest
- 140: portion
- 145: cutouts of Morel Atlas
- 150: portion
- 200: exemplary comparison
- 210: portion
- 220: portion
- 230: portion
- 240: portion
- 300: exemplary view
- 310: region of interest
- 400: exemplary TDI
- 410: tracks
- 500: exemplary (manual) annotation
- 510: DWI
- 520: TDI
- 530: potential thalamic nuclei
- 600: U-net architecture
- 610: encoder
- 620: decoder
- 630: input image
- 635: encoder step
- 640: encoder step
- 645: encoder step
- 650: encoder step
- 655: encoder step
- 660: decoder step
- 665: decoder step
- 670: decoder step
- 675: decoder step
- 680: output segmentation map
- 700: workflow
- 710: step
- 720: step
- 730: step
- 800: workflow
- 810: step
- 820: step
- 830: step
- 840: step
- 900: apparatus
- 910: obtaining unit
- 920: preprocessing unit
- 930: training unit
- 1000: apparatus
- 1010: obtaining unit
- 1020: preprocessing unit
- 1030: providing unit
- 1040: segmenting unit

## Claims

1. A computer-implemented method (700) for training an artificial intelligence model for segmenting a thalamic nucleus in a radiology image, comprising:
obtaining (710) a plurality of radiology images comprising a representation of thalamic nuclei;
preprocessing (720) the obtained plurality of radiology images, wherein the preprocessing comprises generating a plurality of track density images, TDI, (400) based on the generated plurality of radiology images; and
training (730) the artificial intelligence model based on at least a first portion of the generated plurality of TDIs (400).

2. The computer-implemented method of claim 1,
wherein the obtaining of the plurality of radiology images comprises:
obtaining the radiology images for patients of different age groups and ethnicities, preferably at least for 250 patients.

3. The computer-implemented method of any of the preceding claims, further comprising:
validating the trained artificial intelligence model with at least a second portion of the plurality of TDIs (400), wherein the second portion of the TDIs (400) is not used for training the artificial intelligence model.

4. A computer-implemented method (800) for segmenting a thalamic nucleus in a radiology image, comprising:
obtaining (810) a radiology image comprising a representation of the thalamic nucleus;
preprocessing (820) the obtained radiology image, wherein the preprocessing comprises generating a track density image, TDI, (400) based on the obtained radiology image;
providing (830) the generated TDI (400) to a trained artificial intelligence model, wherein the trained artificial intelligence model is trained for segmenting thalamic nuclei in a radiology image; and
segmenting (840), using the trained artificial intelligence model, the thalamic nucleus in the TDI (400).

5. The computer-implemented method of any of the claims 1 - 4,
wherein the obtained plurality of radiology images or the obtained radiology image is a diffusion weighted image, DWI, (510) preferably using a high angular resolution diffusion imaging, HARDI, sequence.

6. The computer-implemented method of claim 5,
wherein obtaining the plurality of radiology images or obtaining the radiology image further comprises:
obtaining the plurality of radiology images or the radiology image for at least 45 diffusion directions, preferably with a diffusion-related b-value of at least 3000 s/mm².

7. The computer-implemented method of any of the claims 5 or 6,
wherein the preprocessing further comprises:
modeling a diffusion depicted in the DWI (510) by means of a spherical harmonic function, wherein the spherical harmonic function is, preferably, at least of order 8.

8. The computer-implemented method of any of the preceding claims, wherein the preprocessing further comprises:
setting a field of view, FOV, (310) in the obtained plurality of radiology images to minimize a TDI (400) generation time.

9. The computer-implemented method of any of the preceding claims,
wherein the TDI (400) comprises at least 15 million tracks, preferably with an isotropic voxel resolution of 0.3mm to 0.4mm voxel side length.

10. The computer-implemented method of any of the preceding claims, wherein the preprocessing further comprises:
segmenting thalamic nuclei in the generated plurality of TDIs (400), preferably manually by a radiologist.

11. The computer-implemented method of any of the preceding claims,
wherein the artificial intelligence model comprises a convolutional neural network, CNN, in a U-net architecture (600).

12. The computer-implemented method of any of the claims 4 - 11,
wherein the trained artificial intelligence model is trained according to the computer-implemented method of any of the claims 1 - 3 or any of the claims 5 - 11 when backreferenced to any of the claims 1 - 3.

13. A trained artificial intelligence model for segmenting thalamic nuclei in a radiology image, wherein the trained artificial intelligence model is trained according to the computer-implemented method of any of the claims 1 - 3 or any of the claims 5 -11 when backreferenced to any of the claims 1 - 3.

14. A computer-implemented apparatus (900) for training an artificial intelligence model for segmenting a thalamic nucleus in a radiology image, comprising:
an obtaining unit for obtaining (910) a plurality of radiology images comprising a representation of thalamic nuclei;
a preprocessing unit for preprocessing (920) the obtained plurality of radiology images, wherein the preprocessing comprises generating a plurality of track density images, TDI, (400) based on the generated plurality of radiology images; and
a training unit for training (930) the artificial intelligence model based on at least a first portion of the generated plurality of TDIs (400).

15. A computer program product comprising instructions which, when the program is executed by a computer, cause the computer to carry out the computer-implemented method of one of claims 1 - 12.
